(19) Europäisches Patentamt
European Patent Office
Office européen des brevets

(11) Publication number: **0 052 510 B2**

(12) # NEW EUROPEAN PATENT SPECIFICATION

(45) Date of publication of the new patent specification: **19.10.94**

(51) Int. Cl.[5]: **A61K 9/50**, A61K 9/52

(21) Application number: **81305426.9**

(22) Date of filing: **17.11.81**

The file contains technical information submitted after the application was filed and not included in this specification

(54) **Microencapsulation of water soluble polypeptides.**

(30) Priority: **18.11.80 US 207864**

(43) Date of publication of application: **26.05.82 Bulletin 82/21**

(45) Publication of the grant of the patent: **27.08.86 Bulletin 86/35**

(45) Mention of the opposition decision: **19.10.94 Bulletin 94/42**

(84) Designated Contracting States: **AT BE CH DE FR GB IT LI LU NL SE**

(73) Proprietor: **SYNTEX (U.S.A.) INC.**
**3401 Hillview Avenue**
**Palo Alto California 94303 (US)**

(72) Inventor: **Kent, John Scott**
**10120 Lockwood Drive**
**Cupertino California 95014 (US)**
Inventor: **Sanders, Lynda Mary**
**765 San Antonio Road 65**
**Palo Alto California 94303 (US)**
Inventor: **Lewis, Danny Harvey**
**312 Jackson Circle**
**Gardendale Alabama 35071 (US)**
Inventor: **Tice, Thomas Robert**
**1305 Overland Drive**
**Birmingham Alabama 35216 (US)**

(74) Representative: **Armitage, Ian Michael et al**
**MEWBURN ELLIS**
**York House**
**23 Kingsway**
**London WC2B 6HP (GB)**

**EP 0 052 510 B2**

(56) References cited:

| | |
|---|---|
| EP-A- 42 753 | EP-A- 49 628 |
| EP-A- 0 021 234 | CH-A- 615 662 |
| DE-A- 2 051 580 | DE-A- 2 625 843 |
| FR-A- 2 400 904 | FR-A- 2 400 950 |
| FR-B- 2 070 153 | GB-A- 1 351 409 |
| GB-A- 1 434 694 | GB-A- 2 035 182 |
| US-A- 3 736 646 | US-A- 3 755 558 |
| US-A- 3 773 919 | US-A- 3 824 227 |
| US-A- 3 826 796 | US-A- 3 835 108 |
| US-A- 3 880 991 | US-A- 3 887 699 |
| US-A- 3 892 723 | US-A- 3 896 105 |
| US-A- 3 991 766 | US-A- 4 010 125 |
| US-A- 4 010 196 | US-A- 4 011 312 |
| US-A- 4 018 726 | US-A- 4 024 121 |
| US-A- 4 138 344 | US-A- 4 148 871 |
| US-A- 4 211 769 | US-A- 4 234 571 |
| ZA-A- 718 150 | |

Luzzi: J.Pharma. Sci., 59 (10), Page 1373, October 1970, pages 1367-1376.

Yolles et al. Controlled Release of Biologically Active Agents", Ed.Tanquary A.C. & Lacey R.E. 1974, pages 177-193

"Polymeric Delivery Systems", Kostelnik R.J. ed., Midl. Macromol. Monogr., 5, 1978, pages 175-196. Langer et al.

"Sustained and Controlled Release Drug Delivery Systems ", Robinson J.R. ed., 1979, pages 123-209. Lee et al.

"Controlled Release of Bioactive Materials", Baker R. ed. Academic Press, New York, 1980, pages 45-60 (Petersen); 83-98 (Langer); 177-187 (Rhine) and 189-212 (Gupta)

Langer: "Controlled Release of Macromolecules", Chemtech, pages 98-105, February 1982

Chang: Journ. Bioengineering, 1, pages 25-31, 1976

Yolles et al., J.Pharma. Sci., 64, pages 348-9, 1975

Beck et al., Am. J. Obstet. Gynecol., 135(3), pages 419-426, October 1979

Beck et al., Fertility and Sterility, 31(5), pages 545-551, May 1979

Beck et at., Research Frontiers in Fertility Regulation, 1(1), July 1980

D.H.Lewis et al. (SRI), 7th International Symposium on Controlled Release of Bioactive Materials, Ft. Lauderlade, Florida, July 28-30th. 1980, pages 129-131

Wise et al., in "Drug Carriers in Biology and Medicine", Gregory Gregoriadis ed., Academic Press-New York-, pages 237-270, 1979

Kent et al., in "Long-Acting Contraceptive Delivery Systems", ed. Zatuchni et al., Harper & Row -Philadelphia- , pages 169-179, 1983

Vickery, J. Steroid Bioechem, 23, pages 779-791

Sanders et al., Journal of Controlled Release, 2, pages 187-195, 1985

Sanders et al., in "LHRH and its analogues", Labrie, Belanger and Du Pont. Eds., pages 53-62, 1984

Ezan et al., Regulatory Peptides, 14, pages 155-167, 1986

Schally et al., The Prostate, 4, pages 545-552, 1983

Schally et al., Research Frontiers in Fertility Regulation, 2(5) July 1983

J.Steroid Biochem, 11, pages 449-445 (1979) (Benagiano)

J.Pharm.Sci., 68, pages 1534-1538 (1979) (Pitt)

Schally et al., Proc. Natl. Acad. Sci., 81, pages 5845-5848, September 1984

Sanders et al., J.Pharm. Sci. 75(4), pages 356-360, April 1986

Chang, Can.J.Physiol. Pharmacol., 44, pages 115-128 (1966)

Brizzolara and Lawter: Pharmaceutical Research, 3(5) 1986 (Supplement)

M.Vert, in "CRC Critical Reviews in Therapeutic Drug Carrier Systems", 2 (3) 1986, pages 311-312

Sanders et al.: J.Pharm.Sci., 73(9) 1984, pages 1294-1297

Herbig, in "Encyc. of Chem. Tech.", Band

13.2 (1967) pages 436-456

Gesetz zur neuordnung des Arzneimittelrechts mit Arzneimittelgesetz, 1976, pages 73-77

Nash, J.Steroid Biochem., 6, 909-915

Gresser, Contraception 17(3), 253-266

Wise, in "Polymeric Delivery Systems" (cf M3); pages 75-89

Miller, J.Biomed.Mater, Res., 11, 711-719, (1977)

Schwope, Life Sci., 17, 1877-1886

Bergquist, The Lancet, 2, 215-6, (1979)

Vickery, "The Endocrine Soc. 61st Ann. Meeting", 1979, Abstract 441

Langer, Nature, 263, 197-200, (1976)

## Description

This invention relates to pharmaceutical microcapsule compositions having sustained release characteristics where the active agent is a water-soluble polypeptide which is a luteinizing hormone/releasing hormone, or analogue thereof, useful for affecting the reproduction function in mammals.

There are a number of publications that disclose combinations of polymers and drugs designed to give sustained or delayed release of drugs. For example U.S. Patent 3,773,919 discloses controlled drug release compositions in which the core comprises a drug, stated to include water-soluble antibiotic polypeptides encapsulated in polylactide/glycolide copolymers as well as similar such polymers.

Microencapsulation for sustained release of enzymes, hormones, vaccines and other biologicals is discussed in a paper by Chang, Thomas, J. Bioeng., Vol. 1, pp 25-32, 1976. Several examples of water-soluble protein encapsulations using polylactic acid are disclosed therein, particularly asparaginase and insulin compositions.

Polylactic acid polymers, polylactide/glycolide copolymers and polyglycolic acid polymers and related materials have been used for making surgical elements, incorporating a medicament and demonstrating slow release properties. See for example U.S. Patents 3,991,776: 4,118,470; 4.076,798.

The invention relates to a pharmaceutical composition designed for sustained release of an effective amount of drug over an extended period of time prepared in microcapsule form wherein the composition comprises:

at least one water soluble polypeptide which is a naturally occurring luteinizing hormone-releasing hormone (LH-RH), a synthetically prepared material of the same type or synthetically prepared analogues of naturally occurring LH-RH which act in some manner on the anterior pituitary gland to affect the release of luteinizing hormone (LH) and follicular stimulating hormone (FSH);

optionally, at least one polymer hydrolysis modifying agent selected from organic acids, acid salts, neutral salts and basic salts; and a biocompatible, biodegradable encapsulating polymer which is a poly-(lactide coglycolide) copolymer; the lactide/glycolide molar ratio of the copolymer, its molecular weight, the capsule diameter, and the polymer hydrolysis modifying agent (if present), being such that the composition exhibits sustained release of an effective amount of the polypeptide over a period of at least one month.

Said composition will release a daily amount of said polypeptide effective for maintaining an hormonally related condition over a predetermined period of time. The specific hormonally related condition bound to the use of said polypeptide is the control of fertility and physiological effects related thereto.

The polymer hydrolysis modifying agents which may be optionally present in the composition may decrease or increase the rate of polymer hydrolysis. They have a low molecular weight and are non-toxic.

The invention also relates to a process for preparing the above pharmaceutical composition, comprising:

dispersing an aqueous solution containing the polypeptide, and optionally a polymer hydrolysis modifying agent, in a halogenated organic solvent containing said encapsulating polymer;

adding to the dispersion a coacervation agent; and

collecting the microcapsules from this solution.

Naturally occurring LH-RH peptides are produced in the hypothalmic region of the brain and control the reproductive cycle of mammals by acting on the anterior pituitary gland to affect release of luteinizing hormone (LH) and follicular stimulating hormone (FSH) which in turn act on the gonads to stimulate the synthesis of steroid hormones and to stimulate gamete maturation. The pulsatile release of LH-RH thereby controls the reproductive cycle in mammals. Additionally, LH-RH has effects in placenta, in releasing HCG, and directly on the gonads. Agonist analogs of LH-RH are useful for the control of fertility by two mechanisms of action. Low doses of LH-RH analogues can stimulate ovulation and are useful in the treatment of hypothalmic and ovulatory infertility. Additionally they can be used for hypogonadal conditions and impotence, and to stimulate spermatogenesis and androgen production in the male. Paradoxically, larger doses of highly potent and long-lasting analogues of LH-RH have an opposite effect and block ovulation in the female and suppress spermatogenesis in the male. Related to these effects is a suppression of normal circulating levels of sexual steroids of gonadal origin, including reduction in accessory organ weight in the male and female. In domestic animals this paradoxical effect promotes weight gain in a feed-lot situation, stimulates abortion in pregnant animals and in general, acts as a chemical sterilant. A full list of the paradoxical high dose effects is set out in European Patent application EP-A-21234.

There is also the group of LH-RH analogues termed antagonists. These polypeptides have the paradoxical effect shown by LH-RH agonists but at low dose levels relative to naturally occurring LH-RH. Such compounds are to be included within the scope of this invention.

The natural hormone releasing hormone LH-RH is a decapeptide comprised of naturally occurring amino acids (which have the L-configuration except for the achiral amino acid glycine). Its sequence is as follows:

(pyro)Glu-His-Trp-Ser-Tyr-Gly-Leu-Arg-Pro-Gly-$NH_2$.

Many analogues of this natural material have been studied. The beneficial effectiveness of these analogues has been varied. The most significant modification where agonists are concerned is obtained by changing the 6-position residue from Gly to a D-amino acid, for example, D-Ala, D-Leu, D-Phe or D-Trp. Antagonist activity can be best realized by substituting the naturally occurring 2-position His amino acid residue with with a D-amino acid residue. These analogues show increased activity relative to LH-RH.

In addition to modifications position 6, increased agonist activity may be obtained by the following modifications: modifying position 10 to afford a nonapeptide as an alkyl-, cycloalkyl- or fluoroalkyl- amine, or by replacing Gly-$NH_2$ by an $\alpha$-azaglycine amide; substituting N-methyl-leucine for leucine in position 7; replacing tryptophan in position 3 by 3-(1-naphthyl)-L-alanine; substituting the position 5 tyrosine residue with phenylalanine or 3-(1-pentafluorophenyl)-L-alanine; and the substitution at position 6 of unnatural D-amino acid residues containing two or more carbocyclic (or perhydroaryl) rings or a phenyl (or cyclohexyl) ring which is highly alkyl substituted. These specific compounds represent some of the more useful fertility affecting LH-RH type polypeptides which have been developed to date. This is not intended to be an exhaustive or exclusive list of all such compounds which which have been made or which can or may be made. They are simply set out to illustrate the type of compounds which are the subject of this invention. Any and all of them can be interchangeably substituted into the compositions of this invention.

The compounds of specific interest herein are those from the last mentioned group wherein the 6-position of the naturally occurring LH-RH material is replaced with a specific unnatural D-amino residue containing lipophilic carbocyclic residues, particularly residues containing two or more highly alkyl substituted carbocyclic aryl (or perhydroaryl) rings or a phenyl (or cyclohexyl) ring. These particular polypeptides are the subject of European Patent application EP-A-21234, and are prepared in accordance with the procedures set forth therein.

More specifically the polypeptides of particular interest in this invention are nonapeptides and decapeptides of the formula:

(pyro)Glu-His-V-Ser-W-X-Y-Arg-Pro-Z (I)

and the pharmaceutically acceptable salts there wherein:

V is tryptophyl, phenylalanyl or 3-(1-naphthyl)-L-alanyl;

W is tyrosyl, phenyalanyl or 3-(1-pentafluorophenyl)-L-alanyl;

X is a D-amino acid residue

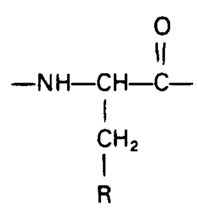

wherein R is

(a) a carbocyclic aryl-containing radical selected from naphthyl, anthryl, fluorenyl, phenanthryl, biphenyl, benzhydryl and phenyl substituted with three or more straight chain lower alkyl groups; or

(b) a saturated carbocyclic radical selected from cyclohexyl substituted with three or more straight chain lower alkyl groups, perhydronaphthyl, perhydrobiphenylyl, perhydro-2,2-diphenylmethyl and adamantyl;

Y is leucyl, Isoleucyl, nor-leucyl or N-methyl-leucyl;

Z is glycinamide or -NH-$R_1$, wherein

$R_1$ is lower alkyl, cycloalkyl, fluoro lower alkyl or

$$-NH-\overset{O}{\overset{\|}{C}}-NH-R^2$$

$R_2$ is hydrogen or lower alkyl.

Preferred compounds of this invention are those wherein X is 3-(2-naphthyl)-D-alanyl or 3-(2,4,6-trimethylphenyl)-D-alanyl; Z is glycinamine; V is tryptophyl or phenylalanyl; W is tyrosyl and Y is leucyl or N-methyl-leucyl.

Particularly preferred compounds are:

(pyro)Glu-His-Trp-Ser-Tyr-3-(2-naphthyl)-D-alanyl-Leu-Arg-Pro-Gly-$NH_2$,
(pyro)Glu-His-Trp-Ser-Tyr-3-(2-naphthyl)-D-alanyl-n-methyl-Leu-Arg-Pro-Gly-$NH_2$,
(pyro)Glu-His-Phe-Ser-Tyr-3-(2-naphthyl)-D-alanyl-Leu-Arg-Pro-Gly-$NH_2$,
(pyro)Glu-His-Trp-Ser-Tyr-3-(2,4,6-trimethylphenyl)-D-alanyl-Leu-Arg-Pro-Gly-$NH_2$,
(pyro)Glu-His-Trp-Ser-Tyr-3-(2-naphthyl)-D-alanyl-Leu-Arg-Pro-NHEt,
(pyro)Glu-His-Trp-Ser-Tyr-3-(2-naphthyl)-D-alanyl-N-methyl-Leu-Arg-Pro-NHEt,

and their pharmaceutically acceptable salts.

Especially preferred is (pyro)Glu-His-Trp-Ser-Tyr-3-(2-naphthyl)-D-alanyl-Leu-Arg-Pro-Gly-$NH_2$ and its pharmaceutically acceptable salts.

As set forth above and for convenience in describing these compounds, the conventional abbreviation for the various amino acids are used as generally accepted in the peptide art as recommended by the IUPAC-IUB Commission on Biochemical Nomenclature, Biochemistry, 11, 1726 (1972) and represent the L-amino acids with the exception of the achiral amino acids in the 6-position designated by X. All peptide sequences mentioned herein are written according to the generally accepted convention whereby the N-terminal amino acid is on the left and the C-terminal amino acid is on the right. The abbreviation "Et" is monovalent ethane.

As used herein, the term "pharmaceutically acceptable salts" refer to the salts that retain the desired biological activity of the parent compound and do not impart any undesired toxicological effects. Examples of such salts can be found in European Patent application EP-A-21234, noted above.

As used herein the term "lower alkyl" refers to a straight or branched chain saturated hydrocarbon group having from 1 to 4 carbon atoms such as, for example, methyl, ethyl, n-propyl, isopropyl, n-butyl, isobutyl, sec-butyl, and tert-butyl; the term "cycloalkyl group" refers to a cyclic saturated hydrocarbon group having from 3 to 6 carbon atoms, for example, cyclopropyl, cyclobutyl, cyclopentyl and cyclohexyl; the term "fluoro lower alkyl" refers to a lower alkyl group wherein one or more hydrogen atoms are replaced by fluorine, such as, for example, trifluoromethyl, pentafluoroethyl and 2,2,2-trifluoroethyl.

As used herein "naphthyl" is inclusive of 1- and 2-naphthyl; "anthryl" is inclusive of 1-, 2- and 9-anthryl; "fluoroenyl" is inclusive of 2-, 3-, 4-, and 9-fluoroenyl; "phenanthryl" is inclusive of 2-, 3- and 9-phenanthryl; and "adamantyl" is inclusive of 1- and 2-adamantyl.

As used herein the phrase "fertility affecting polypeptide" should be understood to mean any naturally occurring LH-RH polypeptide, synthetically prepared material of the same type or synthetically prepared analogues of naturally occurring LH-RH polypeptides which act in some manner on the anterior pituitary gland to affect the release of luteinizing hormone (LH) and follicular stimulating hormone (FSH); and in particular those polypeptides which inhibit ovulation or are useful for treating endometriosis in a female mammalian subject or are useful for treating benign prostatic hypertrophy and inhibiting spermatogenesis in a male mammalian subject.

The compositions of this invention will contain the hormonally active polypeptides in varying amounts depending upon the effect desired. Treatment of infertility requires a low level of drug, while prevention of fertility and related effects requires a large dose relative to the activity of naturally occurring LH-RH. For the agonist fertility control it is expedient to prepare microcapsules which will release the drug at such a rate that the subject will receive between about 0.01 and 100 $\mu$g/kg body weight per day, preferably between 0.1 and 5.0 $\mu$g/kg body weight per day.

The compositions of this invention are formulated to contain the polypeptide in an amount which may vary between 0.01 and 40.0 weight % of the polymer used for encapsulation. Preferably the peptide will be present in the amount between 0.1 to 10.0 weight %.

The amount of drug placed in a particular formulation depends not only on the desired daily dose but also on the number of days that dose level is to be maintained. While this amount can be calculated empirically the actual dose delivered is a function of the degradation characteristics of the encapsulating polymer. Therefore the % weight of drug stated represent amounts which, when taken in conjunction with a

particular polymer provide the desired release profile.

Optionally, certain chemicals which affect the rate of polymer hydrolysis may be dissolved in the aqueous solution containing the polypeptide before it is encapsulated by the polymer excipient. These chemicals are called polymer hydrolysis modifying agents. When present, these compounds may increase or decrease the rate at which the drug is released from the microcapsules. This effect is independent of a particular polymer composition or size.

Four types of chemicals may be used to realize this effect, for example, organic acids, acidic neutral or basic salts. Low molecular weight mono and dicarboxylic acids such as acetic acid, tartaric acid, citric acid, gluconic acid, oxalic acid, ascorbic acid, succinic acid, their salts may be used. Basic salts may be, for example, ammonium sulfate, ammonium chloride, ammonium nitrate and sodium bisulphate. Neutral salts effective herein include metal halides such as, for example, sodium chloride, potassium chloride, sodium bromide, potassium bromide, calcium chloride and magnesium chloride. Basic salts include such salts as sodium carbonate, potassium carbonate, trisodium phosphate and tripotassium phosphate. Of these compounds it is most preferred to use either citric acid, sodium chloride or sodium carbonate. Combinations of these compounds will achieve the desired effect but the compositions described herein contain only one of these agents in a particular composition.

When present the hydrolysis modifying agent will be added in an amount between 0.1 and 20% by weight of the polymer but preferably it will be present in the amount of 5 to 10%.

The biocompatible, biodegradable encapsulating polymer which is used in this invention is a poly-(lactide coglycolide) polymer.

Of course, the polymer must be non-toxic to the host and must be of such composition that it is degradable by the body into metabolic products that have no deleterious or untoward effects on the body. The polymer must also be capable of forming microcapsules containing water-soluble drugs.

A number of particular polymers have been developed which meet these criteria. See, for example U.S. Patent No. 3,773,919.

The copolymers of the invention are derived from the condensation of lactic acid and glycolic acid.

The acid units from which the excipients are prepared may be the optically active (D- and L-) forms or optically inactive (DL-, racemic) forms. For example, lactic acid, whether it is the principle polymer component or the comonomer component, can be present as D-lactic acid, L-lactic acid or DL-lactic acid.

It is most preferred to use lactic acid as the principle monomer with glycolic acid as the comonomer.

The term polylactide is used to designate the general glass of polymers which can be prepared from one or more of the preferred monomers listed above and includes those instances where a single alpha hydroxy acid or lactone is the only monomer in the polymer. For the most preferred polymers those wherein the excipients are prepared solely from the lactic acid monomer or where lactic acid is the principle monomer and glycolic acid is the comonomer are termed poly(lactide-co-glycolide) copolymers.

The combinations of prefered monomer and comonomer which can be prepared are numerous but the most effective excipients are those polymers prepared from lactic acid alone or lactic acid and glycolic acid wherein the glycolic acid is present as a comonomer in a molar ratio of 100:0 to 40:60. It is most preferred to use a poly(lactide-co-glycolide) copolymer having a molar ratio between about 75:25 and 50:50.

Poly(lactide-co-glycolide) polymers may range in size from 20,000 to 100,000 in molecular weight, stated as an average. The molecular weight of a particular copolymer is independent of its monomeric makeup. For example, a 50:50 copolymer can have a molecular weight which falls anywhere within this range. Therefore polymers can be varied both as to their monomer composition and as well as their molecular weight and be within the scope of this invention.

For the purposes of this invention the relative molecular weight of a particular polymer vis-a-vis a second polymer is stated in terms of its inherent viscosity in a particular solvent and at a particular temperature. The viscosity of a particular polymer is measured in a capillary viscometer using chloroform or hexafluoroisopropanol at 30 °C. The results are stated in terms of deciliters/g (dl/g). There is a direct correlation between inherent viscosity and molecular weight.

A method for the preparation of polylactide polymers can be found in U.S. Patent 3,773,919 and reference is made thereto for the preparation of the such polymers.

Preparation of the microcapsules using any combination of the various peptides, polymer hydrolysis modifying agents or encapsulating polymer excipients noted above parallels the basic technique set out in U.S. Patent 3,773,919. A full description of the procedure used herein can be found in that document.

In brief, the procedure involves dissolving the polymer in an halogenated hydrocarbon solvent, dispersing the aqueous drug solution in this polymer-solvent solution, and adding some agent which is soluble in the halogenated hydrocarbon solvent but is a non-solvent for the encapsulating excipient. The addition of the non-solvent, called a coacervation agent, causes the excipient to precipitate out of the

halogenated hydrocarbon solvent onto the dispersed water droplets, thereby encapsulating the polypeptide. For example, a poly(lactide-co-glycolide) is dissolved in methylene chloride. An aqueous solution of polypeptide is then stirred into the solvent-polymer solution to form a water-in-oil emulsion. A second solvent-miscible material such as a silicone oil, is added slowly with stirring to precipitate the excipient which coats the dispersed water droplets to give microcapsules.

Halogenated organic solvents which may be used are most of the C1 to C4 halogenated alkanes such as, for example, methylene chloride, ethylene dichloride, ethylene chloride and 2,2,2-trichloroethane.

Coacervation agents may be any solvent miscible polymeric, mineral oil or vegetable oil compounds which are non-solvents for the encapsulating polymers. There may be used, for example, silicone oil, peanut oil, soybean oil, corn oil, cotton seed oil, coconut oil, linseed oil, and mineral oils.

After being formed, the microcapsules are washed and hardened with an alkane organic solvent, washed with water, washed with an aqueous non-ionic surfactant solution, and then dried at room temperature under vacuum.

Microcapsules may range in diameter from about 1 to 500 μm, depending upon the techniques employed. For this invention it is preferred to have the microcapsule diameter be between 5 and 200 μm.

The prepared microcapsules may be administered to a subject by any means or route desired. However the most effacious route is parenteral administration by injection, most preferably subcutaneously or intramuscularly.

If the capsules are to be administered by injection they may first be suspended in some non-toxic suspending vehicle. The exact make up of these injectable microcapsule suspensions will depend upon the amount of drug to be administered, the suspending capacity of the suspending agent and on the volume of solution which can be injected at a particular site or in a particular subject.

The compositions of this invention exhibit sustained release of the encapsulated compounds over extended periods of time. This time period may range from one month to 3 years depending on the composition of the encapsulating excipient, its molecular weight, the diameter of the capsule, and the presence of a polymer hydrolysis modifying agent in the core. Preferably the release time will be about 1 to 24 months.

The following examples illustrate the compositions and processes of this invention.

Example I

This example describes the procedure for preparing a microcapsules composition wherein the polypeptide is

(pyro)Glu-His-Trp-Ser-Tyr-3-(2-naphthyl)-D-alanyl-Leu-Arg-Pro-Gly-$NH_2$,

(D-Nal(2)$^6$ LH-RH) present in an amount of 1.4% by weight, no polymer hydrolysis modifying agent is present, and the excipient is a 50:50% molar ratio poly(lactide-co-glycolide) copolymer having an inherent viscosity in hexafluoroisopropanol of 0.38 dl/g at 30 °C.

Excipient, 4 g, were dissolved in 196 g of methylene chloride. This solution was placed in a 300 ml resin kettle equipped with a true-bore stirrer having a 63 mm. Teflon (Trade Mark) turbine impeller driven by a Fisher "Steadi-Speed" (Trade Mark) motor. In a 1-dram glass vial was dissolved 0.0571 g of polypeptide in 1.34 g of deionized water. This solution was added to the resin kettle. During this addition, the dilute polymer solution was stirred at 3200 RPM to form a water-in-oil emulsion. With continued stirring at that rate, 80 ml of silicone oil was added at the rate of 4.0 ml/min by means of a peristaltic pump. The silicone oil caused the polymer to phase separate, and deposit as droplets of solvent-swollen polymer onto the surface of the water-polypeptide microdroplets. These solvent-swollen polymer droplets then coalesced to form a continuous film around the water-polypeptide microdroplets. The microcapsules were then hardened by pouring the contents of the resin kettle into a beaker containing 2000 ml of heptane. This mixture was stirred at 1000 RPM for 30 minutes with a stainless-steel impeller. The heptane-methylene chloride-silicone oil solution was removed by filtering the solution, employing a Buchner funnel and Whatman β41 filter paper. The microcapsules were then washed repeatedly with 100-ml aliquots of heptane to insure complete removal of the silicone oil. The microcapsules were then washed with deionized water followed by a wash with a 1% aqueous solution of Tween 20 (Trade Mark). and dried at room temperature under vacuum. Microcapsules obtained from this preparation were determined to have diameters ranging in size from 10 to 40 μm.

The polypeptide containing microcapsules, whose preparation is described in the above paragraph, were suspended in a suspending vehicle and administered as a single subcutaneous injection to female

Sprague-Dawley rats and female rhesus monkeys. The length of estrous suppression was calculated against the percentage of animals showing suppression.

The results of the monkey study are given in Table I below. Each data line represents one subject. The injected dose was as stated in the Table. Microcapsules were prepared as stated in Example I using that LH-RH analogue and a 50:50% molar ratio copolymer (PLA:PGA) having an inherent viscosity of 0.38 dl/g in hexafluoroisopropanol at 30 °C at a 1.4% peptide to polymer ratio. The microcapsule's diameter ranged from 10 to 40 µm.

TABLE I

| Effect of D-Nal(2)$^6$ LHRH released from PLA:PGA microspheres on ovulation in rhesus monkeys | | | | |
|---|---|---|---|---|
| Animal No. | Dose | Before | Intermenstrual interval | |
| | | | During | After treatment |
| 1 | - | 25 | 30 | 28 |
| 2 | - | 28 | 27 | 26, 29 |
| 3 | 1 mg D-Nal(2)$^6$ | 30 | 67 | 27 |
| 4 | 1 mg D-Nal(2)$^6$ | 24 | 83 | 27 |

A single 300 µg dose of D-Nal(2)$^6$ LH-RH micro-encapsulated at 1.4% peptide to polymer with a 50:50% molar ratio poly(lactide-co-glycolide) having a diameter ranging in size from 10-40 µm (inherent viscosity in hexafluoroisopropanol-0.38 dl/g) which had been suspended in a suspending agent (composition given in Example III) was injected subcutaneously in 10 mature female Sprague-Dawley rats. Estrous was determined by daily vaginal smear analysis. All rats showed estrous suppression through day 24 post dosing. At day 25, 40% showed estrous. By day 27 estrous was observed in all animals.

Example II

Table II sets out several examples of polypeptide containing microcapsules wherein the following parameters were varied: molecular weight, stated as inherent viscosity; stir rate; addition rate of silicone oil; and the amount of silicone oil added. The polypeptide encapsulated here is the same as set out in Example I. The preparation techniques described in Example I were used to prepare these materials, except as note for the stirring rates and silicone oil addition rates.

TABLE II

| Batch | Excipient's inherent viscosity, dl/g | Lactide:Glycolide mole ratio | Polymer (g) | Peptide (g) | Silicone oil: Am't added (ml) | Silicone oil: Rate added (ml/min) | Stir rate RPM | Capsule size μm |
|---|---|---|---|---|---|---|---|---|
| A | 0.38[1] | 50:50 | 2.0 | 0.0263 | 40.0 | 4.0 | 3000 | 10—30 |
| B | 0.38[1] | 50:50 | 2.0 | 0.0297 | 135.0 | 2.0 | 1000 | 45—90 |
| C | 1.52[1] | 50:50 | 2.0 | 0.0253 | 40.0 | 4.0 | 3000 | 80—160 |

[1] Inherent viscosity in hexafluoroisopropanol at 30°C.
In each of the above batches the following solvents and amounts used:
to dissolve the peptide—0.67 ml of deionized water;
encapsulation solution—98 ml of methylene chloride.

Example III

The following describes a formulation for parenteral injection of polypeptide-containing microcapsules prepared according to the methods disclosed herein.

Microcapsules containing the polypeptide

(pyro)Glu-His-Trp-Ser-Tyr-3-(2-naphthyl)-D-alanyl-Leu-Arg-Pro-Gly-$NH_2$

in a concentration of 1.0% by weight and wherein the excipient polymer was poly(lactide-co-glycolide) having a molar ratio of 50:50% and an inherent viscosity of 0.38 dl/g in hexafluoroisopropanol at 30 °C were suspended in the following solution:

| | |
|---|---|
| Na CMC | 0.5% |
| NaCl | 0.8% |
| Benzyl alcohol | 0.9% |
| Tween 80 (Trade Mark) | 0.1% |
| Purified water | q.s. 100% |

For example, 330 mg of microcapsules were suspended in 5.5 ml to provide an injectable dose of 300 µg of peptide per 0.5 ml of injectable suspension.

## Claims

### Claims for the following Contracting States : BE, CH, DE, FR, GB, IT, LI, LU, NL, SE

1. A pharmaceutical composition designed for sustained release of an effective amount of drug over an extended period of time prepared in microcapsule form wherein the composition comprises:
   at least one water soluble polypeptide which is a naturally occurring luteinizing hormone-releasing hormone (LH-RH), a synthetically prepared material of the same type or synthetically prepared analogues of naturally occurring LH-RH which act in some manner on the anterior pituitary gland to affect the release of luteinizing hormone (LH) and follicular stimulating hormone (FSH);
   optionally, at least one polymer hydrolysis modifying agent selected from organic acids, acid salts, neutral salts and basic salts; and
   a biocompatible, biodegradable encapsulating polymer which is a poly(lactide-co-glycolide) copolymer;
   the lactide/glycolide molar ratio of the copolymer, its molecular weight, the capsule diameter, and the polymer hydrolysis modifying agent (if present), being such that the composition exhibits sustained release of an effective amount of the polypeptide over a period of at least one month.

2. A composition of claim 1 wherein said polypeptide is a nonapeptide or a decapeptide analogue of LH-RH having the formula

   (pyro)Glu-His-V-Ser-W-X-Y-Arg-Pro-Z

   and the pharmaceutically acceptable salts thereof wherein:
   V is tryptophyl, phenylalanyl or 3-(1-naphthyl)-L-alanyl;
   W is tyrosyl, phenylalanyl or 3-(1-pentafluorophenyl)-L-alanyl;
   X is a D-amino acid residue

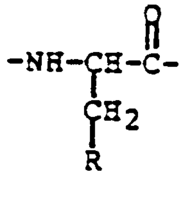

   wherein R is
   (a) a carbocyclic aryl-containing radical selected from naphthyl, anthryl, fluorenyl, phenylanthryl, biphenylyl, benzhydryl and phenyl substituted with three or more straight chain $C_{1-4}$ alkyl groups; or

(b) a saturated carbocyclic radical selected from cyclohexyl substituted with three or more straight chain $C_{1-4}$ alkyl groups, perhydronaphthyl, perhydro- biphenylyl, perhydro-2,2-diphenylmethyl and adamantyl;

Y is leucyl, isoleucyl, nor-leucyl or N-methylleucyl;

Z is glycinamide or -NH-$R_1$, wherein $R_1$ is $C_{1-4}$ alkyl, $C_{3-6}$ cycloalkyl, fluoro $C_{1-4}$ alkyl or

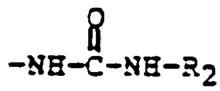

$R_2$ is hydrogen or $C_{1-4}$ alkyl.

**3.** A composition of claim 2 having a polymer which is a poly(lactide-co-glycolide) copolymer wherein the copolymer comprises lactide-glycolide in a molar ratio of between 100:0 and 40:60; and wherein the copolymer has an average molecular weight between about 20,000 and 100,000.

**4.** A composition of claim 3 wherein said polypeptide is present in an amount of between 0.01 and 40.0 weight % of the polymer; and said hydrolysis modifying agent is present in an amount of between 1 and 15 weight % of the polymer.

**5.** A composition of claim 3 or claim 4 having a polypeptide wherein:

V is tryptophyl or phenylalanyl;

W is tyrosyl;

X is 3-(2-naphthyl)-D-alanyl or 3-(2,4,6-trimethylphenyl)-D-alanyl;

Y is leucyl or N-methyl-leucyl; and

Z is glycinamide or NHEt;

said hydrolysis modifying agent is citric acid, ammonium chloride, sodium chloride or sodium carbonate; and

said polymer comprises lactide-co-glycolide in a molar ratio of between 75:25 and 50:50.

**6.** A composition of claim 5 wherein said polypeptide is present in an amount of 0.1 to 10.0 weight %;

said hydrolysis modifying agent is present in an amount of 5 to 10 weight %; and

said polymer comprises lactide-co-glycolide in a molar ratio of 50:50.

**7.** A composition of any one of claims 1 to 6 wherein said polypeptide is (pyro)Glu-His-Trp-Ser-Tyr-3-(2-naphthyl)-D-alanyl-Leu-Arg-Pro-Gly-$NH_2$ or a pharmaceutically acceptable acid salt thereof.

**8.** A composition according to claim 1, wherein the polypeptide is an analogue of natural LH-RH, in which modification comprises the 6-position residue changed from Gly to a D-amino acid.

**9.** A composition according to claim 8, wherein the D-amino acid is D-Ala, D-Leu, D-Phe, or D-Trp.

**10.** A composition according to claim 9, wherein the D-amino acid is D-Leu.

**11.** A composition according to claim 9, wherein the D-amino acid is D-Trp.

**12.** A composition according to any one of claims 8 to 11, wherein the 10-position is modified to afford a nonapeptide as an alkyl-, cycloalkyl- or fluoroalkyl-amine.

**13.** A composition according to any one of claims 8 to 11, wherein the Gly-$NH_2$ is replaced by an $\alpha$-azaglycine amide.

**14.** A composition according to any one of claims 8 to 11, wherein N-methyl-leucine is substituted for leucine in position 7.

**15.** A composition of any one of claims 7 to 14 wherein the copolymer comprises lactide-glycolide in a molar ratio of between 100:0 and 40:60.

16. A composition of any one of claims 7 to 15 wherein said polypeptide is present in an amount of between 0.01 and 40.0 weight % of the polymer.

17. A composition of any one of claims 7 to 16 wherein said hydrolysis modifying agent is present in an amount of between 0.1 and 20 weight %.

18. A composition of any one of claims 7 to 17 wherein said polypeptide is present in an amount of 0.1 to 10.0 weight %.

19. A composition of any one of claims 7 to 18 wherein said copolymer comprises lactide-glycolide in a molar ratio of from 75:25 to 40:60.

20. A composition according to any one of claims 1 to 19 wherein said copolymer comprises lactide-glycolide in a molar ratio of about 50:50.

21. A composition of any one of the preceding claims in the form of injectable particles ranging in size from about 1 to 500 μm.

22. A composition of any of claims 1 to 21 which are dispersed in a pharmaceutically acceptable carrier suitable for parenteral administration.

23. A process for preparing a composition of any one of the preceding claims comprising:
dispersing an aqueous solution containing the polypeptide, and optionally a polymer hydrolysis modifying agent, in a halogenated organic solvent containing said encapsulating polymer;
adding to the dispersion a coacervation agent; and
collecting the microcapsules from this solution.

**Claims for the following Contracting State : AT**

1. A process for preparing a pharmaceutical composition designed for sustained release of an effective amount of drug over an extended period of time prepared in microcapsule form wherein the process comprises:
dispersing an aqueous solution containing a water-soluble polypeptide which is a naturally occurring luteinizing hormone-releasing hormone (LH-RH), a synthetically prepared material of the same type or synthetically prepared analogues of naturally occurring LH-RH which act in some manner on the anterior pituitary gland to affect the release of luteinizing hormone (LH) and follicular stimulating hormone (FSH), and optionally a polymer hydrolysis modifying agent which is an organic acid, acid salt, neutral salt or basic salt, in a halogenated organic solvent containing a biocompatible, biodegradable encapsulating polymer which is a poly(lactide-co-glycolide) copolymer;
adding to the dispersion a coacervation agent; and collecting the microcapsules from this solution;
the lactide/glycolide molar ratio of the copolymer, its molecular weight, the capsule diameter, and the polymer hydrolysis modifying agent (if present), being such that the composition exhibits sustained release of an effective amount of the polypeptide over a period of at least one month.

2. A process of claim 1 wherein said polypeptide is a nonapeptide or a decapeptide analogue of LH-RH having the formula

(pyro)Glu-His-V-Ser-W-X-Y-Arg-Pro-Z

and the pharmaceutically acceptable salts thereof wherein:,
V is tryptophyl, phenylalanyl or 3-(1-naphthyl)-L-alanyl;
W is tyrosyl, phenylalanyl or 3-(1-pentafluorophenyl)-L-alanyl;
X is a D-amino acid residue

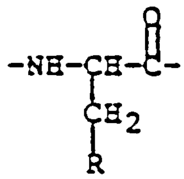

wherein R is

(a) a carbocyclic aryl-containing radical selected from naphthyl, anthryl, fluorenyl, phenylanthryl, bibhenylyl, benzhydryl and phenyl substituted with three or more straight chain $C_{1-4}$ alkyl groups; or

(b) a saturated carbocyclic radical selected from cyclohexyl substituted with three or more straight chain $C_{1-4}$ alkyl groups, perhydronaphthyl, perhydrobiphenylyl, perhydro-2,2-diphenylmethyl and adamantyl;

Y is leucyl, isoleucyl, nor-leucyl or N-methylleucyl;

Z is glycinamide or $-NH-R_1$, wherein $R_1$ is $C_{1-4}$ alkyl, $C_{3-6}$ cycloalkyl, fluoro $C_{1-4}$ alkyl or

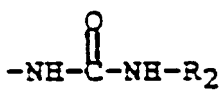

$R_2$ is hydrogen or $C_{1-4}$ alkyl.

**3.** A process of claim 2 using a polymer which is a poly(lactide-co-glycolide) copolymer wherein the copolymer comprises lactide-glycolide in a molar ratio of between 100:0 and 40:60; and wherein the copolymer has an average molecular weight between about 20,000 and 100,000.

**4.** A process of claim 3 wherein said polypeptide is present in an amount of between 0.01 and 40.0 weight % of the polymer; and said hydrolysis modifying agent is present in an amount of between 1 and 15 weight % of the polymer.

**5.** A process of claim 3 or claim 4 using a polypeptide wherein:

V is tryptophyl or phenylalanyl;

W is tyrosyl;

X is 3-(2-naphthyl)-D-alanyl or 3-(2,4,6-trimethylphenyl)-D-alanyl;

Y is leucyl or N-methyl-leucyl; and

Z is glycinamide or NHEt; said hydrolysis modifying agent is citric acid, ammonium chloride, sodium chloride or sodium carbonate; and

said polymer comprises lactide-co-glycolide in a molar ratio of between 75:25 and 50:50.

**6.** A process of claim 5 wherein said polypeptide is present in an amount of 0.1 to 10.0 weight %;

said hydrolysis modifying agent is present in an amount of 5 to 10 weight %; and

said polymer comprises lactide-co-glycolide in a molar ratio of 50:50.

**7.** A process of any one of claims 1 to 6 wherein said polypeptide is (pyro)Glu-His = Trp-Ser-Tyr-3-(2-naphthyl)-D-alanyl-Leu-Arg-Pro-Gly-$NH_2$ or a pharmaceutically acceptable salt thereof.

**8.** A process according to claim 1, wherein the polypeptide is an analogue of natural LH-RH, in which modification comprises the 6-position residue changed from Gly to a D-amino acid.

**9.** A process according to claim 8, wherein the D-amino acid is D-Ala, D-Leu, D-Phe, or D-Trp.

**10.** A process according to claim 9, wherein the D-amino acid is D-Leu.

**11.** A process according to claim 9, wherein the D-amino acid is D-Trp.

**12.** A process according to any one of claims 8 to 11, wherein the 10-position is modified to afford a nonapeptide as an alkyl-, cycloalkyl- or fluoroalkyl-amine.

**13.** A process according to any one of claims 8 to 11, wherein the Gly-$NH_2$ is replaced by an $\alpha$-azaglycine amide.

**14.** A process according to any one of claims 8 to 11, wherein N-methyl-leucine is substituted for leucine in position 7.

**15.** A process according to any one of claims 7 to 14 wherein the copolymer comprises lactide-glycolide in a molar ratio of between 100:0 and 40:60.

**16.** A process of any one of claims 7 to 15 wherein said polypeptide is present in an amount of between 0.01 and 40.0 weight % of the polymer.

**17.** A process of any one of claims 7 to 16 wherein said hydrolysis modifying agent is present in an amount of between 0.1 and 20 weight %.

**18.** A process of any one of claims 7 to 17 wherein said polypeptide is present in an amount of 0.1 to 10.0 weight%.

**19.** A process of any one of claims 7 to 18 wherein said copolymer comprises lactide-glycolide in a molar ratio of from 75:25 to 40:60.

**20.** A process according to any one of claims 1 to 19 wherein said copolymer comprises lactide-glycolide in a molar ratio of about 50:50.

**21.** A process of any one of the preceding claims wherein the product is in the form of injectable particles ranging in size from about 1 to 500 $\mu$m.

**22.** A process of any of claims 1 to 21 wherein the product composition is dispersed in a pharmaceutically acceptable carrier suitable for parenteral administration.

**Patentansprüche**
**Patentansprüche für folgende Vertragsstaaten : BE, CH, DE, FR, GB, IT, LI, LU, NL, SE**

**1.** Pharmazeutische Zusammensetzung für die verzögerte Freigabe einer wirksamen Menge eines Arzneistoffes über eine verlängerte Zeitspanne, hergestellt in Mikrokapselform, worin die Zusammensetzung umfaßt:
mindestens ein wasserlösliches Polypeptid, bei dem es sich um ein natürlich vorkommendes Luteinisierungshormon-Releasinghormon (LH-RH), ein synthetisch hergestelltes Material des gleichen Typs oder synthetisch hergestellte Analoga von natürlich vorkommendem LH-RH handelt, die auf die gleiche Weise auf den Hypophysenvorderlappen einwirken, um die Freisetzung von Luteinisierungshormon (LH) und Follikel-stimulierendem Hormon (FSH) zu beeinflussen;
gegebenenfalls mindestens ein Polymer-Hydrolysemodifizierungsmittel, ausgewählt aus organischen Säuren, sauren Salzen, neutralen Salzen und basischen Salzen; und
ein biokompatibles, bioabbaubares Verkapselungspolymer, das ein Poly(lactid-co-glykolid)-Copolymer ist;
wobei das Lactid/Glykolid-Molverhältnis des Copolymers, dessen Molekulargewicht, der Kapseldurchmesser und das Polymer-Hydrolysemodifizierungsmittel (falls vorhanden) derart sind, daß die Zusammensetzung eine verzögerte Freigabe einer wirksamen Menge des Polypeptids über eine Zeitspanne von mindestens einem Monat aufweist.

**2.** Zusammensetzung nach Anspruch 1, worin es sich bei dem Polypeptid um ein Nonapeptid- oder ein Decapeptid-Analogon von LH-RH mit der Formel

(pyro)Glu-His-V-Ser-W-X-Y-Arg-Pro-Z

und die pharmazeutisch verträglichen Salze davon handelt, worin:

V Tryptophyl, Phenylalanyl oder 3-(1-Naphthyl)-L-alanyl ist;

W Tyrosyl, Phenylalanyl oder 3-(1-Pentafluorphenyl)-L-alanyl ist;

X ein D-Aminosäurerest

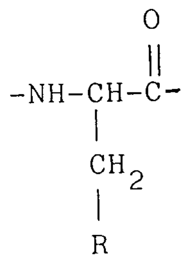

ist, worin R

(a) ein carbocyclischer Aryl-haltiger Rest, ausgewählt aus Naphthyl, Anthryl, Fluorenyl, Phenylanthryl, Biphenylyl, Benzhydryl und Phenyl, substituiert mit drei oder mehr geradkettigen $C_{1-4}$-Alkylgruppen, oder

(b) ein gesättigter carbocyclischer Rest, ausgewählt aus Cyclohexyl, substituiert mit drei oder mehr geradkettigen $C_{1-4}$-Alkylgruppen, Perhydronaphthyl, Perhydrobiphenylyl, Perhydro-2,2-diphenylmethyl und Adamantyl, ist;

Y Leucyl, Isoleucyl, Nor-leucyl oder N-Methylleucyl ist;

Z Glycinamid oder -NH-$R_1$ ist, worin $R_1$ $C_{1-4}$-Alkyl, $C_{3-6}$-Cycloalkyl, Fluor-$C_{1-4}$-alkyl oder

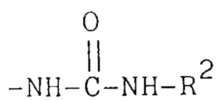

ist;

$R_2$ Wasserstoff oder $C_{1-4}$-Alkyl ist.

**3.** Zusammensetzung nach Anspruch 2 mit einem Polymer, das ein Poly(lactid-co-glykolid)-Copolymer ist, worin das Copolymer Lactid-Glykolid in einem Molverhältnis zwischen 100:0 und 40:60 umfaßt und worin das Copolymer ein durchschnittliches Molekulargewicht zwischen etwa 20000 und 100000 aufweist.

**4.** Zusammensetzung nach Anspruch 3, worin das Polypeptid in einer Menge zwischen 0,01 und 40,0 Gew.-% des Polymers vorhanden ist und das Hydrolysemodifizierungsmittel in einer Menge zwischen 1 und 15 Gew.-% des Polymers vorhanden ist.

**5.** Zusammensetzung nach Anspruch 3 oder Anspruch 4 mit einem Polypeptid, worin:

V Tryptophyl oder Phenylalanyl ist;

W Tyrosyl ist;

X 3-(2-Naphthyl)-D-alanyl oder 3-(2,4,6-Trimethylphenyl)-D-alanyl ist;

Y Leucyl oder N-Methyl-leucyl ist; und

Z Glycinamid oder NHEt ist;

das Hydrolysemodifizierungsmittel Citronensäure, Ammoniumchlorid, Natriumchlorid oder Natriumcarbonat ist und

das Polymer Lactid-Co-Glykolid in einem Molverhältnis zwischen 75:25 und 50:50 umfaßt.

**6.** Zusammensetzung nach Anspruch 5, worin das Polypeptid in einer Menge von 0,1 bis 10,0 Gew.-% vorhanden ist;
das Hydrolysemodifizierungsmittel in einer Menge von 5 bis 10 Gew.-% vorhanden ist und

das Polymer Lactid-Co-Glykolid im Molverhältnis 50:50 umfaßt.

**7.** Zusammensetzung nach irgendeinem der Ansprüche 1 bis 6, worin das Polypeptid (pyro)Glu-His-Trp-Ser-Tyr-3-(2-Naphthyl)-D-alanyl-Leu-Arg-Pro-Gly-$NH_2$ oder ein pharmazeutisch annehmbares Säuresalz davon ist.

**8.** Zusammensetzung nach Anspruch 1, worin das Polypeptid ein Analogon von natürlichem LH-RH ist, in dem die Abwandlung den von Gly zu einer D-Aminosäure veränderten Rest in der Stellung 6 umfaßt.

**9.** Zusammensetzung nach Anspruch 8, worin die D-Aminosäure D-Ala, D-Leu, D-Phe oder D-Trp ist.

**10.** Zusammensetzung nach Anspruch 9, worin die D-Aminosäure D-Leu ist.

**11.** Zusammensetzung nach Anspruch 9, worin die D-Aminosäure D-Trp ist.

**12.** Zusammensetzung nach irgendeinem der Ansprüche 8 bis 11, worin die Stellung 10 abgeändert ist, um ein Nonapeptid als Alkyl-, Cycloalkyl- oder Fluoralkylamin zu ergeben.

**13.** Zusammensetzung nach irgendeinem der Ansprüche 8 bis 11, worin das Gly-$NH_2$ durch ein $\alpha$-Azaglycinamid ersetzt ist.

**14.** Zusammensetzung nach irgendeinem der Ansprüche 8 bis 11, worin Leucin in der Stellung 7 durch N-Methyl-leucin ersetzt ist.

**15.** Zusammensetzung nach irgendeinem der Ansprüche 7 bis 14, worin das Copolymer Lactid-Glykolid in einem Molverhältnis von zwischen 100:0 und 40:60 umfaßt.

**16.** Zusammensetzung nach irgendeinem der Ansprüche 7 bis 15, worin das Polypeptid in einer Menge zwischen 0,01 und 40,0 Gew.-% des Polymers vorhanden ist.

**17.** Zusammensetzung nach irgendeinem der Ansprüche 7 bis 16, worin das Hydrolysemodifizierungsmittel in einer Menge zwischen 0,1 und 20 Gew.-% vorhanden ist.

**18.** Zusammensetzung nach irgendeinem der Ansprüche 7 bis 17, worin das Polypeptid in einer Menge von 0,1 bis 10,0 Gew.-% vorhanden ist.

**19.** Zusammensetzung nach irgendeinem der Ansprüche 7 bis 18, worin das Copolymer Lactid-Glykolid in einem Molverhältnis von 75:25 bis 40:60 umfaßt.

**20.** Zusammensetzung nach irgendeinem der Ansprüche 1 bis 19, worin das Copolymer Lactid-Glykolid in einem Molverhältnis von ungefähr 50:50 umfaßt.

**21.** Zusammensetzung nach irgendeinem der vorangehenden Ansprüche in Form von injizierbaren Teilchen mit einem Größenbereich von etwa 1 bis 500 µm.

**22.** Zusammensetzung nach irgendeinem der Ansprüche 1 bis 21, die in einem für die parenterale Applikation geeigneten pharmazeutisch annehmbaren Träger dispergiert ist.

**23.** Verfahren zur Herstellung einer Zusammensetzung nach irgendeinem der vorangehenden Ansprüche, umfassend:
Dispergieren einer wäßrigen Lösung, die das Polypeptid und gegebenenfalls ein Polymer-Hydrolysemodifizierungsmittel enthält, in einem halogenierten organischen Lösungsmittel, welches das Verkapselungspolymer enthält;
Versetzen der Dispersion mit einem Coazervierungsmittel; und
Sammeln der Mikrokapseln aus der Lösung.

**Patentansprüche für folgenden Vertragsstaat : AT**

1. Verfahren zur Herstellung einer pharmazeutischen Zusammensetzung für die verzögerte Freigabe einer wirksamen Menge eines Arzneistoffes über eine verlängerte Zeitspanne, hergestellt in Mikrokapselform, worin das Verfahren umfaßt:
Dispergieren einer wäßrigen Lösung, enthaltend ein wasserlösliches Polypeptid, bei dem es sich um ein natürlich vorkommendes Luteinisierungshormon-Releasing-hormon (LH-RH), ein synthetisch hergestelltes Material des gleichen Typs oder synthetisch hergestellte Analoga von natürlich vorkommendem LH-RH handelt, die auf die gleiche Weise auf den Hypophysenvorderlappen einwirken, um die Freisetzung von Luteinisierungshormon (LH) und Follikel-stimulierendem Hormon (FSH) zu beeinflussen, und gegebenenfalls ein Polymer-Hydrolysemodifizierungsmittel, das eine organische Säure, ein saures Salz, neutrales Salz oder basisches Salz ist, in einem halogenierten organischen Lösungsmittel, welches ein biokompatibles, bioabbaubares Verkapselungspolymer, das ein Poly(lactid-co-glykolid)-Copolymer ist, enthält;
Versetzen der Dispersion mit einem Coazervierungsmittel; und Sammeln der Mikrokapseln aus der Lösung;
wobei das Lactid/Glykolid-Molverhältnis des Copolymers, dessen Molekulargewicht, der Kapseldurchmesser und das Polymer-Hydrolysemodifizierungsmittel (falls vorhanden) derart sind, daß die Zusammensetzung eine verzögerte Freigabe einer wirksamen Menge des Polypeptids über eine Zeitspanne von mindestens einem Monat aufweist.

2. Verfahren nach Anspruch 1, worin es sich bei dem Polypeptid um ein Nonapeptid- oder ein Decapeptid-Analogon von LH-RH mit der Formel

(pyro)Glu-His-V-Ser-W-X-Y-Arg-Pro-Z

und die pharmazeutisch verträglichen Salze davon handelt, worin:
V Tryptophyl, Phenylalanyl oder 3-(1-Naphthyl)-L-alanyl ist;
W Tyrosyl, Phenylalanyl oder 3-(1-Pentafluorphenyl)-L-alanyl ist;
X ein D-Aminosäurerest

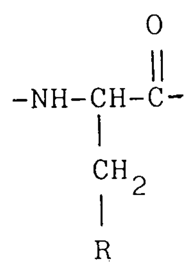

ist, worin R
(a) ein carbocyclischer Aryl-haltiger Rest, ausgewählt aus Naphthyl, Anthryl, Fluorenyl, Phenylanthryl, Biphenylyl, Benzhydryl und Phenyl, substituiert mit drei oder mehr geradkettigen $C_{1-4}$-Alkylgruppen, oder
(b) ein gesättigter carbocyclischer Rest, ausgewählt aus Cyclohexyl, substituiert mit drei oder mehr geradkettigen $C_{1-4}$-Alkylgruppen, Perhydronaphthyl, Perhydrobiphenylyl, Perhydro-2,2-diphenylmethyl und Adamantyl, ist;
Y Leucyl, Isoleucyl, Nor-leucyl oder N-Methylleucyl ist;
Z Glycinamid oder -NH-$R_1$ ist, worin $R_1$ $C_{1-4}$-Alkyl, $C_{3-6}$-Cycloalkyl, Fluor-$C_{1-4}$-alkyl oder

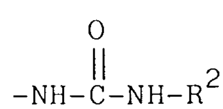

ist;
$R_2$ Wasserstoff oder $C_{1-4}$-Alkyl ist.

**3.** Verfahren nach Anspruch 2 unter Verwendung eines Polymers, das ein Poly(lactid-co-glykolid)-Copolymer ist, worin das Copolymer Lactid-Glykolid in einem Molverhältnis zwischen 100:0 und 40:60 umfaßt und worin das Copolymer ein durchschnittliches Molekulargewicht zwischen etwa 20000 und 100000 aufweist.

**4.** Verfahren nach Anspruch 3, worin das Polypeptid in einer Menge zwischen 0,01 und 40,0 Gew.-% des Polymers vorhanden ist und das Hydrolysemodifizierungsmittel in einer Menge zwischen 1 und 15 Gew.-% des Polymers vorhanden ist.

**5.** Verfahren nach Anspruch 3 oder Anspruch 4 unter Verwendung eines Polypeptids, worin:
V Tryptophyl oder Phenylalanyl ist;
W Tyrosyl ist;
X 3-(2-Naphthyl)-D-alanyl oder 3-(2,4,6-Trimethylphenyl)-D-alanyl ist;
Y Leucyl oder N-Methyl-leucyl ist; und
Z Glycinamid oder NHEt ist;
das Hydrolysemodifizierungsmittel Citronensäure, Ammoniumchlorid, Natriumchlorid oder Natriumcarbonat ist und
das Polymer Lactid-Co-Glykolid in einem Molverhältnis zwischen 75:25 und 50:50 umfaßt.

**6.** Verfahren nach Anspruch 5, worin das Polypeptid in einer Menge von 0,1 bis 10,0 Gew.-% vorhanden ist;
das Hydrolysemodifizierungsmittel in einer Menge von 5 bis 10 Gew.-% vorhanden ist und
das Polymer Lactid-Co-Glykolid im Molverhältnis 50:50 umfaßt.

**7.** Verfahren nach irgendeinem der Ansprüche 1 bis 6, worin das Polypeptid (pyro)Glu-His-Trp-Ser-Tyr-3-(2-Naphthyl)-D-alanyl-Leu-Arg-Pro-Gly-$NH_2$ oder ein pharmazeutisch annehmbares Säuresalz davon ist.

**8.** Verfahren nach Anspruch 1, worin das Polypeptid ein Analogon von natürlichem LH-RH ist, in dem die Abwandlung den von Gly zu einer D-Aminosäure veränderten Rest in der Stellung 6 umfaßt.

**9.** Verfahren nach Anspruch 8, worin die D-Aminosäure D-Ala, D-Leu, D-Phe oder D-Trp ist.

**10.** Verfahren nach Anspruch 9, worin die D-Aminosäure D-Leu ist.

**11.** Verfahren nach Anspruch 9, worin die D-Aminosäure D-Trp ist.

**12.** Verfahren nach irgendeinem der Ansprüche 8 bis 11, worin die Stellung 10 abgeändert ist, um ein Nonapeptid als Alkyl-, Cycloalkyl- oder Fluoralkylamin zu ergeben.

**13.** Verfahren nach irgendeinem der Ansprüche 8 bis 11, worin das Gly-$NH_2$ durch ein $\alpha$-Azaglycinamid ersetzt ist.

**14.** Verfahren nach irgendeinem der Ansprüche 8 bis 11, worin Leucin in der Stellung 7 durch N-Methylleucin ersetzt ist.

**15.** Verfahren nach irgendeinem der Ansprüche 7 bis 14, worin das Copolymer Lactid-Glykolid in einem Molverhältnis von zwischen 100:0 und 40:60 umfaßt.

**16.** Verfahren nach irgendeinem der Ansprüche 7 bis 15, worin das Polypeptid in einer Menge zwischen 0,01 und 40,0 Gew.-% des Polymers vorhanden ist.

**17.** Verfahren nach irgendeinem der Ansprüche 7 bis 16, worin das Hydrolysemodifizierungsmittel in einer Menge zwischen 0,1 und 20 Gew.-% vorhanden ist.

**18.** Verfahren nach irgendeinem der Ansprüche 7 bis 17, worin das Polypeptid in einer Menge von 0,1 bis 10,0 Gew.-% vorhanden ist.

**19.** Verfahren nach irgendeinem der Ansprüche 7 bis 18, worin das Copolymer Lactid-Glykolid in einem Molverhältnis von 75:25 bis 40:60 umfaßt.

**20.** Verfahren nach irgendeinem der Ansprüche 1 bis 19, worin das Copolymer Lactid-Glykolid in einem Molverhältnis von ungefähr 50:50 umfaßt.

**21.** Verfahren nach irgendeinem der vorangehenden Ansprüche, worin das Produkt in Form von injizierbaren Teilchen mit einem Größenbereich von etwa 1 bis 500 $\mu$m ist.

**22.** Verfahren nach irgendeinem der Ansprüche 1 bis 21, worin die Produktzusammensetzung in einem für die parenterale Applikation geeigneten pharmazeutisch annehmbaren Träger dispergiert ist.

**Revendications**
**Revendications pour les Etats contractants suivants : BE, CH, DE, FR, GB, IT, LI, LU, NL, SE**

**1.** Composition pharmaceutique conçue pour la libération soutenue d'une quantité efficace de médicament pendant une période prolongée, préparée sous forme de microcapsules, qui comprend :

au moins un polypeptide hydrosoluble qui est une hormone libérant l'hormone lutéinisante (LH-RH) d'origine naturelle, une matière du même type préparée par synthèse ou des analogues préparés par synthèse de l'hormone LH-RH d'origine naturelle, qui agissent d'une certaine manière sur la glande hypophysaire antérieure pour effectuer la libération de l'hormone lutéinisante (LH) et de l'hormone folliculo-stimulante (FSH) ;

à titre facultatif, au moins un agent modificateur d'hydrolyse de polymère choisi entre des acides organiques, des sels acides, des sels neutres et des sels basiques ; et

un polymère d'encapsulation biocompatible et biodégradable qui est un copolymère du type poly-(lactide-co-glycolide) ;

le rapport molaire lactide/glycolide du copolymère, son poids moléculaire, le diamètre des capsules et l'agent modificateur d'hydrolyse de polymère (s'il est présent) étant tels que la composition présente une libération prolongée d'une quantité efficace du polypeptide sur une période d'au moins un mois.

**2.** Composition suivant la revendication 1, dans laquelle ledit polypeptide est un analogue nonapeptidique ou décapeptidique de l'hormone LH-RH, répondant à la formule

(pyro)Glu-His-V-Ser-W-X-Y-Arg-Pro-Z

et ses sels pharmaceutiquement acceptables, formule dans laquelle

V est un groupe tryptophyle, phénylalanyle ou 3-(1-naphtyl)-L-alanyle :

W est un groupe tyrosyle, phénylalanyle ou 3-(1-pentafluorophényl)-L-alanyle ;

X est un résidu de D-amino-acide de formule

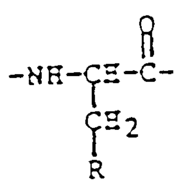

dans laquelle R représente

(a) un radical carbocyclique contenant un groupe aryle, choisi entre les radicaux naphtyle, anthryle, fluorényle, phénylanthryle, biphénylyle, benzhydryle et phényle substitué avec trois ou plus de trois groupes alkyle à chaîne droite en $C_1$ à $C_4$ ; ou

(b) un radical carbocyclique saturé choisi entre un radical cyclohexyle substitué avec trois ou plus de trois groupes alkyle en $C_1$ à $C_4$ à chaîne droite, perhydronaphtyle, perhydrobiphénylyle, perhydro-2,2-diphénylméthyle et adamantyle ;

Y est un groupe leucyle, isoleucyle, norleucyle ou N-méthyl-leucyle ;

Z est un groupe glycinamide ou un groupe -NH-$R_1$, dans lequel
$R_1$ est un groupe alkyle en $C_1$ à $C_4$, cycloalkyle en $C_3$ à $C_6$, fluoralkyle en $C_1$ à $C_4$ ou

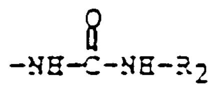

$R_2$ est l'hydrogène ou un groupe alkyle en $C_1$ à $C_4$.

**3.** Composition suivant la revendication 2, contenant un polymère qui est un copolymère du type poly-(lactide-co-glycolide), ce copolymère comprenant des motifs lactide-glycolide dans un rapport molaire compris entre 100:0 et 40:60 ; et ce copolymère ayant un poids moléculaire moyen compris entre environ 20 000 et 100 000.

**4.** Composition suivant la revendication 3, dans laquelle ledit polypeptide est présent en une quantité comprise entre 0,01 et 40,0 % en poids du polymère ; et ledit agent modificateur d'hydrolyse est présent en une quantité comprise entre 1 et 15 % en poids du polymère.

**5.** Composition suivant la revendication 3 ou la revendication 4, contenant un polypeptide dans lequel :
V est un groupe tryptophyle ou phénylalanyle ;
W est un groupe tyrosyle ;
X est un groupe 3-(2-naphtyl)-D-alanyle ou 3-(2,4,6-triméthylphényl)-D-alanyle ;
Y est un groupe leucyle ou N-méthyl-leucyle ; et
Z est un groupe glycinamide ou NHEt ;
ledit agent modificateur d'hydrolyse est l'acide citrique, le chlorure d'ammonium, le chlorure de sodium ou le carbonate de sodium ; et
ledit polymère comprend les motifs lactide-co-glycolide dans un rapport molaire compris entre 75:25 et 50:50.

**6.** Composition suivant la revendication 5, dans laquelle ledit polypeptide est présent en une quantité de 0,1 à 10,0 % en poids ;
ledit agent modificateur d'hydrolyse est présent en une quantité de 5 à 10 % en poids: et
ledit polymère comprend les motifs lactide-co-glycolide dans un rapport molaire de 50:50.

**7.** Composition suivant l'une quelconque des revendications 1 à 6, dans laquelle ledit polypeptide est le (pyro)Glu-His-Trp-Ser-Tyr-3-(2-naphtyl)-D-alanyl-Leu-Arg-Pro-Gly-$NH_2$ ou un sel pharmaceutiquement acceptable de ce composé.

**8.** Composition suivant la revendication 1, dans laquelle le polypeptide est un analogue de l'hormone LH-RH naturelle, dans lequel la modification porte sur le changement du résidu Gly en position 6 en un D-amino-acide.

**9.** Composition suivant la revendication 8, dans laquelle le D-amino-acide est D-Ala, D-Leu, D-Phe ou D-Trp.

**10.** Composition suivant la revendication 9, dans laquelle le D-amino-acide est D-Leu.

**11.** Composition suivant la revendication 9, dans laquelle le D-amino-acide est D-Trp.

**12.** Composition suivant l'une quelconque des revendications 8 à 11, dans laquelle la position 10 est modifiée en alkylamine, cycloalkylamine ou fluoralkylamine pour offrir un nonapeptide.

**13.** Composition suivant l'une quelconque des revendications 8 à 11, dans laquelle le Gly-$NH_2$ est remplacé par un $\alpha$-azaglycine-amide.

**14.** Composition suivant l'une quelconque des revendications 8 à 11, dans laquelle la N-méthyl-leucine remplace la leucine en position 7.

**15.** Composition suivant l'une quelconque des revendications 7 à 14, dans laquelle le copolymère comprend les motifs lactide-glycolide dans un rapport molaire compris entre 100:0 et 40:60.

**16.** Composition suivant l'une quelconque des revendications 7 à 15, dans laquelle ledit polypeptide est présent en une quantité comprise entre 0,01 et 40,0 % en poids du polymère.

**17.** Composition suivant l'une quelconque des revendications 7 à 16, dans laquelle ledit agent modificateur d'hydrolyse est présent en une quantité comprise entre 0,1 et 20 % en poids.

**18.** Composition suivant l'une quelconque des revendications 7 à 17, dans laquelle ledit polypeptide est présent en une quantité de 0,1 à 10,0 % en poids.

**19.** Composition suivant l'une quelconque des revendications 7 à 18, dans laquelle ledit copolymère comprend les motifs lactide-glycolide dans un rapport molaire de 75:25 à 40:60.

**20.** Composition suivant l'une quelconque des revendications 1 à 19, dans laquelle le copolymère comprend les motifs lactide-glycolide dans un rapport molaire d'environ 50:50.

**21.** Composition suivant l'une quelconque des revendications précédentes, sous la forme de particules injectables dont le diamètre va d'environ 1 à 500 µm.

**22.** Composition suivant l'une quelconque des revendications 1 à 21, qui est en dispersion dans un véhicule pharmaceutiquement acceptable propre à l'administration parentérale.

**23.** Procédé de préparation d'une composition suivant l'une quelconque des revendications précédentes, qui consiste :

à disperser une solution aqueuse contenant le polypeptide, et à titre facultatif, un agent modificateur d'hydrolyse de polymère, dans un solvant organique halogéné contenant ledit polymère d'encapsulation ;

à ajouter à la dispersion un agent de coacervation ; et

à recueillir les microcapsules de cette solution.

**Revendications pour l'Etat contractant suivant : AT**

**1.** Procédé de préparation d'une composition pharmaceutique sous forme de microcapsules, conçue pour la libération soutenue d'une quantité efficace de médicament pendant une période prolongée, procédé qui comprend :

la dispersion d'une solution aqueuse contenant un polypeptide hydrosoluble qui est une hormone libérant l'hormone lutéinisante (LH-RH) d'origine naturelle, une matière du même type préparée par synthèse ou des analogues préparés par synthèse de l'hormone LH-RH d'origine naturelle, qui agissent d'une certaine manière sur la glande hypophysaire antérieure pour effectuer la libération de l'hormone lutéinisante (LH) et de l'hormone folliculostimulante (FSH), et à titre facultatif, un agent modificateur d'hydrolyse de polymère qui est un acide organique, un sel acide, un sel neutre ou un sel basique, dans un solvant organique halogéné contenant un polymère d'encapsulation biocompatible et biodégradable qui est un copolymère du type poly(lactide-co-glycolide) ;

l'addition à la dispersion d'un agent de coacervation ; et

la séparation des microcapsules de cette solution ;

le rapport molaire lactide/glycolide du copolymère, son poids moléculaire, le diamètre des capsules et l'agent modificateur d'hydrolyse de polymère (s'il est présent) étant tels que la composition présente une libération prolongée d'une quantité efficace du polypeptide sur une période d'au moins un mois.

**2.** Procédé suivant la revendication 1, dans lequel ledit polypeptide est un analogue nonapeptidique ou décapeptidique de l'hormone LH-RH, répondant à la formule

(pyro)Glu-His-V-Ser-W-X-Y-Arg-Pro-Z

et ses sels pharmaceutiquement acceptables, formule dans laquelle

V est un groupe tryptophyle, phénylalanyle ou 3-(1-naphtyl)-L-alanyle ;

W est un groupe tyrosyle, phénylalanyle ou 3-(1-pentafluorophényl)-L-alanyle ;
X est un résidu de D-amino-acide de formule

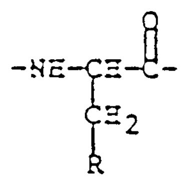

dans laquelle R représente

(a) un radical carbocyclique contenant un groupe aryle, choisi entre les radicaux naphtyle, anthryle, fluorényle, phénylanthryle, biphénylyle, benzhydryle et phényle substitué avec trois ou plus de trois groupes alkyle à chaîne droite en $C_1$ à $C_4$ ; ou

(b) un radical carbocyclique saturé choisi entre un radical cyclohexyle substitué avec trois ou plus de trois groupes alkyle en $C_1$ à $C_4$ à chaîne droite, perhydronaphtyle, perhydrobiphénylyle, perhydro-2,2-diphénylméthyle et adamantyle ;

Y est un groupe leucyle, isoleucyle, norleucyle ou N-méthyl-leucyle ;
Z est un groupe glycinamide ou un groupe -NH-$R_1$, dans lequel
$R_1$ est un groupe alkyle en $C_1$ à $C_4$, cycloalkyle en $C_3$ à $C_6$, fluoralkyle en $C_1$ à $C_4$ ou

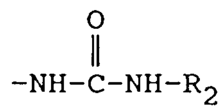

$R_2$ est l'hydrogène ou un groupe alkyle en $C_1$ à $C_4$.

**3.** Procédé suivant la revendication 2, utilisant un polymère qui est un copolymère du type poly(lactide-co-glycolide), qui comprend les motifs lactide-glycolide dans un rapport molaire compris entre 100:0 et 40:60 ; le copolymère ayant un poids moléculaire moyen compris entre environ 20 000 et 100 000.

**4.** Procédé suivant la revendication 3, dans lequel ledit polypeptide est présent en une quantité comprise entre 0,01 et 40,0 % en poids du polymère ; et ledit agent modificateur d'hydrolyse est présent en une quantité comprise entre 1 et 15 % en poids du polymère.

**5.** Procédé suivant la revendication 3 ou la revendication 4, utilisant un polypeptide dans lequel :
V est un groupe tryptophyle ou phénylalanyle ;
W est un groupe tyrosyle ;
X est un groupe 3-(2-naphtyl)-D-alanyle ou 3-(2,4,6-triméthylphényl)-D-alanyle ;
Y est un groupe leucyle ou N-méthyl-leucyle ; et
Z est un groupe glycinamide ou NHEt ;
ledit agent modificateur d'hydrolyse est l'acide citrique, le chlorure d'ammonium, le chlorure de sodium ou le carbonate de sodium ; et
ledit polymère comprend les motifs lactide-co-glycolide dans un rapport molaire compris entre 75:25 et 50:50.

**6.** Procédé suivant la revendication 5, dans lequel ledit polypeptide est présent en une quantité de 0,1 à 10,0 % en poids ;
ledit agent modificateur d'hydrolyse est présent en une quantité de 5 à 10 % en poids; et
ledit polymère comprend les motifs lactide-co-glycolide dans un rapport molaire de 50:50.

**7.** Procédé suivant l'une quelconque des revendications 1 à 6, dans lequel ledit polypeptide est le (pyro)-Glu-His-Trp-Ser-Tyr-3-(2-naphtyl)-D-alanyl-Leu-Arg-Pro-Gly-$NH_2$ ou un sel pharmaceutiquement acceptable de ce composé.

**8.** Procédé suivant la revendication 1, dans lequel le polypeptide est un analogue de l'hormone LH-RH naturelle, dans lequel la modification porte sur le changement du résidu Gly en position 6 en un D-amino-acide.

9. Procédé suivant la revendication 8, dans lequel le D-amino-acide est D-Ala, D-Leu, D-Phe ou D-Trp.

10. Procédé suivant la revendication 9, dans lequel le D-amino-acide est D-Leu.

11. Procédé suivant la revendication 9, dans lequel le D-amino-acide est D-Trp.

12. Procédé suivant l'une quelconque des revendications 8 à 11, dans lequel la position 10 est modifiée en une alkylamine, cycloalkylamine ou fluoralkylamine pour offrir un nonapeptide.

13. Procédé suivant l'une quelconque des revendications 8 à 11, dans lequel le Gly-$NH_2$ est remplacé par un $\alpha$-azaglycine-amide.

14. Procédé suivant l'une quelconque des revendications 8 à 11, dans lequel la N-méthyl-leucine remplace la leucine en position 7.

15. Procédé suivant l'une quelconque des revendications 7 à 14, dans lequel le copolymère comprend les motifs lactide-glycolide dans un rapport molaire compris entre 100:0 et 40:60.

16. Procédé suivant l'une quelconque des revendications 7 à 15, dans lequel ledit polypeptide est présent en une quantité comprise entre 0,01 et 40,0 % en poids du polymère.

17. Procédé suivant l'une quelconque des revendications 7 à 16, dans lequel ledit agent modificateur d'hydrolyse est présent en une quantité comprise entre 0,1 et 20 % en poids.

18. Procédé suivant l'une quelconque des revendications 7 à 17, dans lequel ledit polypeptide est présent en une quantité de 0,1 à 10,0 % en poids.

19. Procédé suivant l'une quelconque des revendications 7 à 18, dans lequel ledit copolymère comprend les motifs lactide-glycolide dans un rapport molaire de 75:25 à 40:60.

20. Procédé suivant l'une quelconque des revendications 1 à 19, dans lequel ledit copolymère comprend les motifs lactide-glycolide dans un rapport molaire d'environ 50:50.

21. Procédé suivant l'une quelconque des revendications précédentes, dans lequel le produit est sous la forme de particules injectables dont les diamètres sont compris dans un intervalle d'environ 1 à 500 $\mu$m.

22. Procédé suivant l'une quelconque des revendications 1 à 21, dans lequel la composition de produit est dispersée dans un véhicule pharmaceutiquement acceptable propre à l'administration parentérale.